# EUROPEAN PATENT APPLICATION

(11) **EP 4 710 981 A1**
(43) Date of publication of application: **18.03.2026**
(21) Application number: 24802866.4
(22) Date of filing: 30.04.2024
(51) Int. Cl.: A61M 25/01

(54) **OPEN TYPE CLAMPING STRUCTURE AND OPEN TYPE STERILE MODULE**

(30) Priority: 06.05.2023 CN 202310504471; 09.05.2023 CN 202310517391; 02.02.2024 CN 202410155803
(71) Applicant: Shenzhen Tonglu Technology Co., Ltd, Shenzhen, Guangdong 518000 (CN)
(72) Inventor: CAO, Yunfei, Shenzhen, Guangdong 518000 (CN); PENG, Zhicong, Shenzhen, Guangdong 518000 (CN); PENG, Xiaohui, Shenzhen, Guangdong 518000 (CN); XIN, Ann, Lincoln Massachusetts (US)
(74) Representative: Fish & Richardson P.C.
(86) International application number: PCT/CN2024/090823
(87) International publication number: WO 2024/230584

(57) **Abstract**

Herein disclosed is a medical clamping apparatus with an openable assembly. The apparatus includes a first tunable assembly having a first lumen and a second tunable assembly having a second lumen that are interconnected to each other in a way that the first and second lumens are extended along the same longitudinal direction and relatively tunable. The first and second lumens are each provided with a first through slot and a second through slot, respectively. Each through slot is longitudinally along respective walls of each lumen, and across from outside of the respective lumen into the respective inside of the respective lumen to allow access of an intracavity medical device to be placed into or taken out of the lumens. Furthermore, in the first tunable assembly, there are two clamping members and two corresponding pressing members that are installed inside the first lumen, with the two clamping members holding the intracavity medical device, wherein when the first tunable assembly is turned relative to the second tunable assembly, the two pressing members force the two clamping members to approach each other and slide into the first lumen.

## Description

### CROSS-REFERENCES TO RELATED APPLICATION

This application claims priority to PCT application No.PCT/CN2024/090823, Pub. No. WO 2024/230584A1, entitled "OPEN TYPE CLAMPING STRUCTURE AND OPEN TYPE STERILE MODULE," filed on April 30, 2024, claiming priority of CN202310504471.5, filed on May 6, 2023, CN202310517391.3 filed on May 9, 2023, and CN202410155803.8, filed on February 2, 2024, the entire content of which is incorporated herein by reference.

### TECHNICAL FIELD

This application relates generally to the field of surgical robots, particularly to a clamping apparatus with an openable assembly, and an openable sterile module for clinically operating intracavity medical devices such as catheters and guidewires.

### BACKGROUND

In the existing practice of surgeries involving the use of intravascular catheters, the surgeon needs to stand near the patient to operate intravascular medical devices such as guidewires, guiding catheters, or angioplasty catheters, inserting them into or withdrawing them from the patient's vascular system. Throughout the procedure, the surgeon is inevitably exposed to radiation from the medical imaging equipment. Although protective measures such as lead aprons are used, the prolonged wearing of lead aprons increases the surgeon's physical exertion, not only raising the risk of hand tremors and affecting the quality of the already inefficient surgery, but also posing significant occupational hazards to surgeons working long-term in radiation environments, resulting in high labor intensity.

Robotic-assisted surgery technology offers advantages such as precision, reliability, fine movements, and minimally invasive procedures, which can greatly enhance surgical accuracy while effectively reducing radiation exposure to surgeons. It has broad development prospects and is a key research direction in the field of surgical robots. Existing surgical robots require corresponding clamping mechanisms to facilitate the advancement, retraction, and/or rotation of intracavity medical devices. However, current designs are cumbersome, costly, and inconvenient to clamp and operate.

### SUMMARY

In view of the shortcomings of the existing arts, there sets forth a disclosure providinga clamping apparatus with an openable assembly and an openable sterile module that facilitatesconvenient clamping and access for medical devices, such as catheters, guidewires, etc. to be placed into, or taken out from, the catheterization.

The present disclosure provides a clamping apparatus with an openable assembly. The apparatus includes a first tunable assembly having a first lumen and a second tunable assembly having a second lumen that are interconnected to each other in a way that the first and second lumens are extended along the same longitudinal direction and relatively tunable. The first and second lumens are each provided with a first through slot and a second through slot, respectively. Each through slot is longitudinally along respective walls of each lumen, and across from outside of the respective lumen into the respective inside of the respective lumen to allow access of an intracavity medical device to be placed into or taken out of the lumens. Furthermore, in the first tunable assembly, there are two clamping members and two corresponding pressing members that are installed inside the first lumen, with the two clamping members holding the intracavity medical device, wherein when the first tunable assembly is turned relative to the second tunable assembly, the two pressing members force the two clamping members to approach each other and slide into the first lumen.

Preferably, the first tunable assembly includes a first notched ring and a sleeve protruding from one side of the first notched ring, and the second tunable assembly includes a second notched ring and a shaft protruding from one side of the second notched ring and rotatably mounted within the sleeve.

Preferably, the first tunable assembly further includes a first central body fixed along the turning axis of the first notched ring, the first central body being provided with two receiving holes for accommodating the two clamping members, the two receiving holes are interconnected with the first lumen.

Preferably, the first central body includes a main body, a left block, and a right block attached together by the first notched ring, wherein the left and right blocks are spaced apart on opposite sides of a notch of the first notched ring, and the two receiving holes are formed between the left block, the right block, and the main body.

Preferably, the main body is configured with a recess to form a longitudinal section crossing the first lumen and the two receiving holes, the longitudinal section being provided with two pivot shafts, and the two pressing members being rotatably mounted on the two pivot shafts.

Preferably, the left block and the right block are respectively provided with positioning caps fitted over the two pivot shafts, positioning the two pressing members between the left block, the right block, and the main body.

Preferably, each of the two clamping members has a through hole interconnected with the corresponding receiving hole. Each pressing member includes a first arm extending into the receiving hole and located within the through hole of the corresponding clamping member and a second arm connected to the first arm, the first arm forming two opposing protrusions abutting against opposite sides of the corresponding through hole.

Preferably, the wall of the first lumen is provided with a first flared portion, a linkage rod is disposed within the second lumen, and a first end of the linkage rod extends out of one end of the second lumen to form a large head accommodated within the first flared portion.

Preferably, the second arms of the two pressing members are each provided with an elongated sliding slot, and the large head is provided with two sliding posts slidably inserted into the elongated sliding slots of the corresponding second arms.

Preferably, the opposite second end of the linkage rod extends out of the opposite end edge of the second lumen and is engaged therewith with the two pressing members being two levers arranged in a snap-fit configuration. When the first tunable assembly rotates relative to the second tunable assembly, the linkage rod moves away from or toward the first tunable assembly along with the second tunable assembly, thereby causing the two pressing members to force the two clamping members to clamp or release the intracavity medical device.

Preferably, the linkage rod is provided with a third lumen and a third through slot extending along the direction of the turning axis, the third through slot extending from the outside to the third lumen to allow communication with the external environment, and the third lumen being interconnected with the first lumen.

Preferably, the clamping apparatus with openable assembly further includes an anti-disengagement member rotatably attached within the third lumen, along a turning axis of the anti-disengagement member, there being provided with a fourth lumen and a fourth through slot extending from the outside to the fourth lumen. The first, third, and fourth lumens are arranged longitudinally along the same straight line and are interconnected. When the first and second tunable assemblies is turned relative to each other and the anti-disengagement member is turned to align the first, second, third, and fourth through slots, allowing the first, third, and fourth lumens to be interconnected with the external environment for placement of the intracavity medical device. When the anti-disengagement member is turned to misalign the fourth through slot with the second and third through slots, disengagement of the intracavity medical device is prevented.

Preferably, the anti-disengagement member includes a twisting portion and a central rod extending from one side of the twisting portion and rotatably inserted into the third lumen.

Preferably, the anti-disengagement member further comprises a sleeve portion extending from one side of the twisting portion. A receiving groove is configured to be formed between the sleeve portion and the central rod to accommodate the opposite second end of the linkage rod, with the sleeve portion being fitted over the second end of the linkage rod.

Preferably, the wall of the first lumen is provided with a second flared portion near the first flared portion, and each of the two clamping members includes an abutting portion located within the second flared portion.

Preferably, each of the two clamping members includes a sliding portion slidably received in the corresponding receiving hole, the through hole of the clamping member being provided in the sliding portion and extending in a direction perpendicular to an axis of the two receiving holes.

Preferably, the first tunable assembly further includes a first support column protruding from an opposite side of the first notched ring, the second tunable assembly further comprises a second support column protruding from an opposite side of the second notched ring. The first lumen and the first through slot extend through the main body, the first support column, and the sleeve, the second lumen and the second through slot extend through the second support column and the shaft, and the notches of the first and second notched rings are respectively aligned with the first and second through slots.

Preferably, the first and second notched rings are configured as driven rotation members for non-contact spatial control/transmission.

Preferably, herein disclosed further includes an open sterile module which includes a housing and the clamping apparatus with openable assembly rotatably attached within the housing. The housing is provided with a placement slot communicating with the interior to allow an intracavity medical device to enter the housing through the placement slot and be clamped by the clamping apparatus, the clamping apparatus as described above.

Preferably, opposite sides of the above housing are respectively provided with a first placement hole and a second placement hole interconnected with the interior of the housing. Both ends of the placement slot extend to the first and second placement holes, and the first lumen of the first tunable assembly and the second lumen of the second tunable assembly are respectively aligned with the first and second placement holes.

Preferably, the open sterile module further includes two bearing seats fixed within the housing. The first and second tunable assemblies are tunably supported on the two bearing seats.

Preferably, a driven movement block is fixed on the housing for non-contact spatial control/transmission.

In summary, the clamping apparatus with openable assembly of the present invention includesa first tunable assembly having a first lumen and a second tunable assembly having a second lumen that are interconnected to each other in a way that the first and second lumens are extended along the same longitudinal direction and relatively tunable. a first through slot and a second through slot, allowing an intracavity medical device to be conveniently inserted into the first and second central holes from the outside for clamping and to be easily removed from the first and second central holes through the first and second through slots. Both clamping and removal are highly convenient, improving surgical efficiency. The open sterile module includes a housing with a placement slot, allowing the intracavity medical device to be conveniently inserted into the housing for clamping by the open clamping structure.

### BRIEF DESCRIPTION OF THE DRAWINGS

In the drawings, each reference numeral generally refers to the same part throughout the different views. Also, the drawings are not necessarily to scale, with emphasis instead generally being placed upon illustrating the principles of the disclosed systems and methods and are not intended as limiting. For clarity, not every component may be labeled in every drawing. In the following description, various embodiments are described with reference to the following drawings.
Fig. 1 is an exploded schematic view of an embodiment of the clamping apparatus with openable assembly according to the present disclosure.
Fig. 2 is a cross-sectional view of the partially assembled clamping apparatus according to the present disclosure.
Fig. 3 is a schematic view of the clamping apparatus with an openable assembly according to the present disclosure.
Fig. 4 is an exploded schematic view of the open clamping device for the sterile module according to the present disclosure.
Fig. 5 is a schematic view of an embodiment of the clamping apparatus with an openable assembly in an open state according to the present disclosure.

### DETAILED DESCRIPTION

The forgoing description of the partially reusable robot apparatus can be used for insertion or removal of catheters, as an example. It should be appreciated that the scope and spirit of this disclosure is not limited to this example. The terms of "robot moving hand" and "moving hand" can be interchangeably used. Similarly for the terms of "robot stationary hand" and "stationary hand".

The forgoing description of the present disclosure is described in detail in conjunction with the accompanying drawings and embodiments. It should be understood that the specific embodiments herein described are only used to explain the present disclosure, not to limit the present disclosure.

In the present disclosure, unless otherwise clearly specified and limited, the terms "installation", "attachment", and "connection" should be understood in a broad sense, for example, it may be a fixed attachment, a detachable connection, or an integral body. It may also be an attachment that can move relatively. It may also be a mechanical connection or an electrical connection. It may be a direct or indirect connection through an intermediary. It may be an internal communication of two components or the interaction relationship between two components. The specific meanings of the above terms used in the present disclosure are to be understood by those skilled in the art according to specific situations.

In the description of the present disclosure, the terms "upper", "lower", "front", "rear", "left", "right", "horizontal", "bottom", "inner", etc. indicate orientation or positional relationship in reference to the corresponding drawings. It is only for the convenience of describing the present disclosure, rather than indicating or implying that the referred device or component must have a specific orientation or must be constructed and operated in a specific orientation, therefore shall not be construed as a limitation of the disclosure.

In addition, the terms "first", "second", etc. are used for descriptive purposes only, and should not be understood as indicating or implying relative importance or implicitly specifying the quantity of the indicated technical features. Thus, a feature defined as "first", "second", etc. may expressly or implicitly include one or more of that kind of features.

The interventional robotic platform of the present disclosure can be used for delivery or withdrawal of, for example, intraluminal medical devices. It should be understood that the term "intraluminal" includes lumens such as natural lumens, panvascular lumens, and organ lumens. The term "intraluminal medical device" may refer to any shape or any kind of catheter, guide wire, guide catheter, angioplasty catheter, or endoscope, various laparoscopes, tube mirrors, etc., including those suitable for vascular intervention, any catheter, guide wire, or consumable device for coronary artery disease, electrophysiology, structural heart disease and other procedures. The guide wires herein include but not limited to peripheral guide wires, coronary guide wires, loach guide wires, contrast guide wires and micro guide wires, etc. for guiding and supporting intracavitary medical devices, and catheters include but not limited to guide catheters, coronary catheters, micro catheters, contrast catheters, intermediate tubes (also known as intermediate catheters), thrombolytic catheters, balloon dilation catheters and ball dilation stent catheters and other diagnostic and therapeutic intracavitary medical devices.

The terms "far" and "front" used in the present disclosure are directionally toward the clinical subject. The term "near" is the directionally away from the subject or to be near the interventional robot. The term "advance" refers to the direction in which the endoluminal medical device is placed into the clinical subject. The term "backward" refers to the direction in which the endoluminal medical device is withdrawn out of the subject.

It should further be noted that, when it is feasible, the embodiments of the present disclosure and various features in the embodiments may be combined with each other, and such combinations are all within the protection scope of the present disclosure.

In the description of the present invention, the term "proximal end" refers to the end closer to the surgeon, and the term "distal end" refers to the end farther from the surgeon; the terms "delivery", "pushing", "advancing", "pulling", or "dragging" refer to the process of moving from a location away from the surgeon towards the surgeon, while the terms "withdrawal" or "retreating" refer to the process of moving from a location near the surgeon away from the surgeon. The terms "horizontal", "vertical", "upper", "lower", "left", "right", "inner", "outer", "therebetween", "between", etc., indicating directions or positional relationships are based on the directions or positional relationships shown in the drawings, and are only for the convenience of describing the present invention and simplifying the description, rather than indicating or implying that the referred device or element must have a specific orientation, be constructed and operated in a specific orientation, and therefore cannot be understood as limiting the present invention. Unless otherwise specified and limited, the terms "connected", "linked", "fixed", "installed" should be understood in a broad sense, for example, it can be a fixed connection, a detachable connection, or an integral connection; it can be a mechanical connection, an electrical connection, or a magnetic connection; it can be directly connected or indirectly connected through an intermediate medium, and it can be the internal communication of two elements or the interaction relationship between two elements. For those of ordinary skill in the art, the specific meanings of the above terms in the present invention can be understood according to specific circumstances.

Additionally, the terms "first", "second", etc., are only used for descriptive purposes and cannot be understood as indicating or implying relative importance or implicitly indicating the number of technical features indicated. Therefore, features defined with "first", "second", etc., may explicitly or implicitly include one or more of such features. In the description of the present invention, "multiple" means more than one, unless otherwise specifically defined.

The surgical execution device and the surgical robot having the same according to the present invention are used for delivering or withdrawing, for example, intravascular medical devices. It should be understood that the term "intravascular" includes natural lumens, pan-vascular lumens, and organ lumens, etc. The term "intravascular medical device" can refer to any shape or type of guidewire, catheter, guiding guidewire, guiding catheter, angioplasty catheter, or endoscope, various laparoscopes, tube scopes, etc., including any guidewire-like or catheter-like consumables or non-consumable instruments suitable for natural lumen, pan-vascular intervention, electrophysiology, structural heart disease, and other surgical procedures. Here, guidewires (and the like) include but are not limited to guiding guidewires, loach guidewires, angiographic guidewires, and micro guidewires for guiding and supporting intravascular medical devices, and catheters (and the like) include but are not limited to guiding catheters, microcatheters, angiographic catheters, intermediate tubes (also known as intermediate catheters), thrombolytic catheters, balloon dilation catheters, and balloon-expandable stent catheters for diagnostic and therapeutic intravascular medical devices. It should be understood that the scope and spirit of the present invention are not limited to these examples of the present invention.

Finally, it should be noted that if there is no conflict, the embodiments of the present invention and the various features in the embodiments can be combined with each other, all within the protection scope of the present invention.

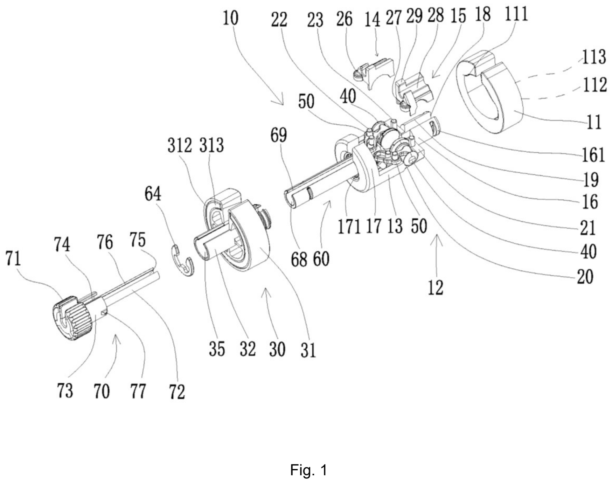

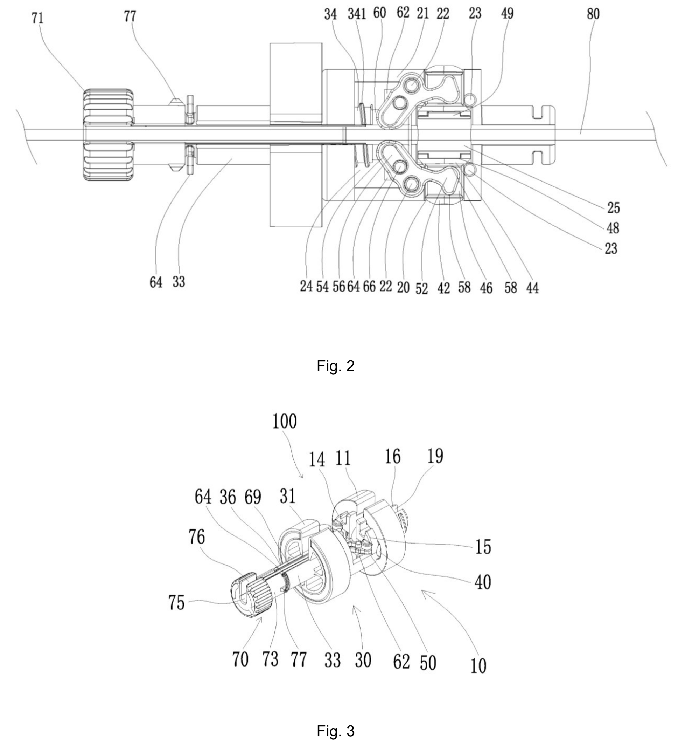

Referring to Figures 1-3, these are schematic diagrams of the first embodiment of the open clamping apparatus of the present invention. For clarity, although some components are omitted in the figures, they remain important. The open clamping apparatus 100 includes a first tunable assembly 10, a second tunable assembly 30, at least two clamping members 40, two pressing members, a linkage rod 60, and an anti-disengagement member 70.

The first tunable assembly 10 includes a first notched ring 11 and a first central body 12 located at the turning axis of the first notched ring 11 (i.e., the turning axis of the first tunable assembly 10). The first central body 12 includes a main body 13, a left block 14, and a right block 15. The main body 13 extends outward on two opposite sides to form a first support column 16 and a sleeve 17. In this embodiment, the first notched ring 11 is detachably fitted over the first central body 12, with the first support column 16 and the sleeve 17 located on opposite sides of the main body 13 and extending in opposite directions. The inner wall of the sleeve 17 forms an internal thread 171. The first central body 12 is provided with a first lumen 18 and a first through slot 19, the first lumen 18 being formed along the extension direction of the turning axis of the first central body 12 (i.e., the turning axis of the first notched ring 11) and extending through the main body 13, the first support column 16, and the sleeve 17. The first through slot 19 extends radially from the outside to the first lumen 18, allowing communication with the external environment. The notch of the first notched ring 11 is aligned with the first through slot 19 to communicate with the first lumen 18, so the notch of the first notched ring 11 can also be considered part of the first through slot 19.

The main body 13, between the first support column 16 and the sleeve 17, is provided with at least two receiving holes 20 corresponding to the two clamping members 40 and is recessed to form a longitudinal section 21 crossing the first lumen 18 and the receiving holes 20. The two receiving holes 20 are aligned and forming a passage with the first lumen 18. In this embodiment, the two receiving holes 20 are located on the same straight line passing through the turning axis of the first central body 12 and perpendicular to the extension direction of the turning axis. The longitudinal section 21 is provided with two pivot shafts 22 and two positioning pins 23 corresponding to the pressing members, the left block 14, and the right block 15. The two pivot shafts 22 and the two positioning pins 23 are aligned similarly to the two receiving holes 20 and are located on opposite sides of the aligned receiving holes 20. In this embodiment, the two pivot shafts 22 and two positioning pins 23 on the longitudinal section 21 are symmetrically arranged relative to the extension direction of the turning axis of the first central body 12. The wall of the first lumen 18, corresponding to the main body 13 between the first support column 16 and the sleeve 17, is provided with adjacent first flared portion 24 and second flared portion 25.

The second tunable assembly 30 includes a second notched ring 31 and a second central body 32 located at the turning axis of the second notched ring 31 (i.e., the turning axis of the second tunable assembly 30). The second central body 32 includes a second support column 33 and a shaft 34 protruding from opposite sides of the second notched ring 31, respectively, extending outward in opposite directions. The second central body 32 is provided with a second lumen 35 and a second through slot 36, the second lumen 35 being formed along the extension direction of the turning axis of the second central body 32 (i.e., the turning axis of the second notched ring 31) and extending through the second support column 33 and the shaft 34. The second through slot 36 extends radially from the outside to the second lumen 35, allowing communication with the external environment. The notch of the second notched ring 31 is aligned with the second through slot 36 to communicate with the second lumen 35, so the notch of the second notched ring 31 can also be considered part of the second through slot 36. The outer wall of the shaft 34 forms an external thread 341 to cooperate with the internal thread 171 of the sleeve 17, allowing the shaft 34 to rotate relative to the sleeve 17, positioning the shaft 34 coaxially within the sleeve 17, achieving rotational connection between the first tunable assembly 10 and the second tunable assembly 30, and aligning the first through slot 19 with the second through slot 36 for interconnection.

Each clamping member 40 includes a sliding portion 42 slidably received in the corresponding receiving hole 20 and an abutting portion 44 formed on the sliding portion 42 and located within the second flared portion 25 of the first lumen 18. The sliding portion 42 is provided with a through hole 46, and the abutting portion 44 has a clamping surface 48. In this embodiment, the extension direction of the through hole 46 (parallel to the extension direction of the turning axis of the first central body 12) is perpendicular to the straight line direction of the two receiving holes 20. To ensure reliable clamping of the intracavity medical device 80 by the clamping surfaces 48 of the two clamping members 40, they may have any shape or be provided with structures to increase friction or clamping force. Additionally, in this embodiment, to prevent damage to the intracavity medical device 80 during clamping, protective pads 49 are attached to the clamping surfaces 48 of the two clamping members 40.

The linkage rod 60 is inserted into the second lumen 35 of the second tunable assembly 30, with its first end extending out of the shaft 34 to form a large head 62, and its opposite second end engaged with the end of the second support column 33 via a structure such as a snap ring 64, allowing the linkage rod 60 to rotate relative to the second tunable assembly 30 but not move axially along the extension direction of the turning axis of the second central body 32. The large head 62 is accommodated within the first flared portion 24 of the first lumen 18 and has a platform 64 provided with two sliding posts 66. The linkage rod 60 is provided with a third lumen 68 and a third through slot 69, the third lumen 68 extending through the entire linkage rod 60 along the extension direction of its turning axis. The third through slot 69 extends through the entire linkage rod 60 along the extension direction of its turning axis and radially from the outside to the third lumen 68, allowing communication with the external environment. Thus, the third lumen 68 is in communication with the first lumen 18, and the third through slot 69 is aligned with the first through slot 19 of the first tunable assembly 10 on the same straight line.

In this embodiment, the two pressing members are bent levers 50, rotatably fixed on the two pivot shafts 22 of the main body 13, positioned on the longitudinal section 21 of the main body 13 in a snap-fit configuration. Each bent lever 50 includes a first arm 52 extending into the receiving hole 20 and located within the through hole 46 of the corresponding clamping member 40 and a second arm 54 connected to the first arm 52, the extension direction of the first arm 52 forming an obtuse angle with the extension direction of the second arm 54. The second arm 54 is provided with an elongated sliding slot 56 corresponding to the sliding post 66 of the linkage rod 60, allowing the sliding post 66 to slide within the elongated sliding slot 56 when the bent lever 50 rotates.

Using the two positioning pins 23, the left block 14 and the right block 15 are fixed to the longitudinal section 21 of the main body 13, and the first notched ring 11 is fitted over the outer periphery of the main body 13, the left block 14, and the right block 15 to assemble them together, forming the first through slot 19 between the notch of the first notched ring 11, the left block 14, the right block 15, the first support column 16, and the sleeve 17 of the main body 13. The two receiving holes 20 are formed between the left block 14, the right block 15, and the main body 13, extending radially from the wall of the first lumen 18 to the outer periphery, i.e., the two receiving holes 20 are located on the same straight line perpendicular to the extension direction of the first lumen 18. Meanwhile, the two bent levers 50 are respectively positioned between the left block 14, the right block 15, and the main body 13. The left block 14 and the right block 15 are respectively provided with positioning caps 26 and 27 fitted over the two pivot shafts 22 to restrict the two bent levers 50 to rotate only around the corresponding pivot shafts 22, with the two positioning pins 23 used to position the left block 14 and the right block 15. To prevent relative movement of the first notched ring 11 with respect to the main body 13, the left block 14, and the right block 15, a locking structure is provided between the left block 14, the right block 15, and the first notched ring 11, such as grooves 28 formed on the left block 14 and the right block 15, and protrusions 111 on the inner side of the first notched ring 11 that engage with the corresponding grooves 28 to restrict relative rotation of the first notched ring 11 with the main body 13, the left block 14, and the right block 15 in both clockwise and counterclockwise directions; as shown, the left block 14 and the right block 15 have step structures 29 to restrict axial movement of the first notched ring 11 along the extension direction of the turning axis of the first central body 12.

The anti-disengagement member 70 includes a twisting portion 71, a central rod 72 extending from one side of the twisting portion 71, and a sleeve portion 73. The sleeve portion 73 is fitted over the central rod 72, forming a receiving groove 74 between the sleeve portion 73 and the central rod 72. The anti-disengagement member 70 is provided with a fourth lumen 75 and a fourth through slot 76. The fourth lumen 75 extends through the entire anti-disengagement member 70 along the extension direction of its turning axis. The fourth through slot 76 extends through the entire anti-disengagement member 70 along the extension direction of its turning axisand radially from the outside to the fourth lumen 75, allowing communication with the external environment. The twisting portion 71 may be provided with anti-slip patterns, as shown. The sleeve portion 73 has a protrusion 77 on its outer periphery.

The central rod 72 of the anti-disengagement member 70 is inserted into the third lumen 68 of the linkage rod 60, with its second end inserted into the receiving groove 74, allowing the anti-disengagement member 70 to be rotatably fixed to the linkage rod 60. Thus, the fourth lumen 75 is in communication with the first lumen 18 and the third lumen 68. By using the twisting portion 71, the central rod 72 can rotate within the third lumen 68, and the sleeve portion 73 can rotate relative to the second end of the linkage rod 60, aligning the fourth through slot 76 of the anti-disengagement member 70 with the third through slot 69 of the linkage rod 60 for interconnection.

To clamp an intracavity medical device 80, the first notched ring 11 (first tunable assembly 10) and the second notched ring 31 (second tunable assembly 30) are rotated relative to each other in opposite directions, i.e., the sleeve 17 of the first tunable assembly 10 rotates relative to the shaft 34 of the second tunable assembly 30, bringing the first notched ring 11 and the second notched ring 31 closer together. Simultaneously, the large head 62 of the linkage rod 60 is driven to gradually approach the two clamping members 40, with the two sliding posts 66 on its platform 64 sliding from the far point (i.e., the end of the second arm 54) to the near point within the two elongated sliding slots 56 of the two bent levers 50, forcing the two bent levers 50 to rotate around the two pivot shafts 22, bringing the two second arms 54 of the two bent levers 50 closer together and moving the two first arms 52 farther apart. When the two first arms 52 move within the through holes 46 of the two clamping members 40, they force the two clamping members 40 to slide within the corresponding receiving holes 20 and separate from each other. In this embodiment, the two first arms 52 each form two opposing protrusions 58 abutting against opposite sides of the corresponding through holes 46, facilitating the movement of the two clamping members 40 toward or away from each other. At this point, the open clamping apparatus is in an open state, with the first through slot 19 of the first tunable assembly 10, the third through slot 69, and the second through slot 36 of the second tunable assembly 30 aligned on the same straight line, and the second through slot 36 of the second tunable assembly 30 in communication with the third through slot 69 of the linkage rod 60. The operator manually rotates the twisting portion 71 of the anti-disengagement member 70 to align its fourth through slot 76 with the third through slot 69 of the linkage rod 60. Thus, the first through slot 19 of the first tunable assembly 10, the second through slot 36 of the second tunable assembly 30, the third through slot 69 of the linkage rod 60, and the fourth through slot 76 of the anti-disengagement member 70 are aligned on the same straight line, allowing the first lumen 18, the third lumen 68, and the fourth lumen 75 to communicate with the external environment, enabling the intracavity medical device 80 to be conveniently inserted through the first through slot 19 (including the notch of the first notched ring 11), the second through slot 36 (including the notch of the second notched ring 31), the third through slot 69, and the fourth through slot 76 into the first lumen 18, the third lumen 68, and the fourth lumen 75. Since the sizes of the first through slot 19, the second through slot 36, the third through slot 69, the fourth through slot 76, the first lumen 18, the third lumen 68, and the fourth lumen 75 can be arbitrarily set, intracavity medical devices 80 of different sizes (e.g., thicknesses) can be accommodated.

The operator manually rotates the twisting portion 71 of the anti-disengagement member 70 to misalign its fourth through slot 76 with the second through slot 36, the third through slot 69, and the first through slot 19, preventing communication between the fourth lumen 75 and the external environment. Then, the sleeve 17 (first notched ring 11) of the first tunable assembly 10 and the shaft 34 (second notched ring 31) of the second tunable assembly 30 are rotated relative to each other in directions opposite to the original rotation, moving the first notched ring 11 and the second notched ring 31 farther apart, driving the large head 62 of the linkage rod 60 to gradually move away from the two clamping members 40, with the two sliding posts 66 on its platform 64 sliding from the near point to the far point within the two elongated sliding slots 56 of the two bent levers 50, forcing the two bent levers 50 to rotate around the two pivot shafts 22, moving the two second arms 54 of the two bent levers 50 farther apart and bringing the two first arms 52 closer together. As the two first arms 52 move within the through holes 46 of the two clamping members 40, their protrusions 58 force the two clamping members 40 to slide within the corresponding receiving holes 20 and approach each other until the clamping surfaces 48 of the two clamping members 40 or their protective pads 49 clamp the intracavity medical device 80. At this point, the open clamping apparatus is in a closed state, and the first notched ring 11 and the second notched ring 31 stop rotating relative to each other in opposite directions. Instead, the first notched ring 11 (first tunable assembly 10) and the second notched ring 31 (second tunable assembly 30) rotate together in the same direction to drive the intracavity medical device 80 to rotate. During rotation, since the anti-disengagement member 70 is fixed to the linkage rod 60, it rotates with the second tunable assembly 30 along with the linkage rod 60, ensuring that even if the second through slot 36 of the second tunable assembly 30 aligns with the third through slot 69 of the linkage rod 60, the intracavity medical device 80 will not accidentally disengage because the fourth through slot 76 of the anti-disengagement member 70 is misaligned with the third through slot 69, preventing communication between the fourth lumen 75 and the external environment, forming a closed space.

To release the intracavity medical device 80, the first notched ring 11 (first tunable assembly 10) and the second notched ring 31 (second tunable assembly 30) are rotated relative to each other in opposite directions again, bringing the shaft 34 closer to the two clamping members 40, and the large head 62 of the linkage rod 60 forces the two bent levers 50 to rotate around the two pivot shafts 22, bringing the two second arms 54 of the two bent levers 50 closer together and moving the two first arms 52 farther apart. Thus, the protrusions 58 of the two first arms 52 abut against the two clamping members 40, gradually moving them apart, causing the clamping surfaces 48 or their protective pads 49 to release the intracavity medical device 80, and aligning the first through slot 19, the second through slot 36, and the third through slot 69 on the same straight line. At this point, the operator manually rotates the twisting portion 71 of the anti-disengagement member 70 to align its fourth through slot 76 with the third through slot 69 for interconnection. The intracavity medical device 80 can then be conveniently removed from the first lumen 18, the third lumen 68, and the fourth lumen 75 through the first through slot 19, the second through slot 36, the third through slot 69, and the fourth through slot 76.

The aforementioned "relative rotation in opposite directions" means that when one of the first notched ring 11 (first tunable assembly 10) and the second notched ring 31 (second tunable assembly 30) rotates clockwise, the other rotates counterclockwise; the aforementioned "rotation in the same direction" means that the first notched ring 11 (first tunable assembly 10) and the second notched ring 31 (second tunable assembly 30) rotate simultaneously in either the clockwise or counterclockwise direction.

Additionally, the first notched ring 11 and the second notched ring 31 each have annular embedding grooves 112 and 312, into which permanent magnet rings 113 and 313 are embedded, making the first notched ring 11 (first tunable assembly 10) and the second notched ring 31 (second tunable assembly 30) magnetic gears for non-contact spatial control/transmission. Specifically, the permanent magnet rings 113 and 313 can be integral or composed of several permanent magnet blocks. When the permanent magnet rings 113 and 313 are integral, adjacent magnetic poles after magnetization are opposite; when composed of several permanent magnet blocks, adjacent permanent magnet blocks have opposite magnetic poles. Regarding magnetic gears and their non-contact spatial control/transmission, refer to the surgical execution module and surgical robot described in Chinese Patent Application 202310565522.5, the surgical execution device and surgical robot with the same described in Chinese Patent Application 202310237481.7, the surgical robot device and its operation method described in Chinese Patent Application 202210803401.5, and the surgical robot described in Chinese Patent Application 202211105526.7, the entire contents of which are incorporated into the present invention. The figures in this embodiment show discontinuous magnetic gears with notches; in other embodiments, continuous magnetic gears may be used. Therefore, essentially, the magnetic gears formed by the first notched ring 11 (first tunable assembly 10) and its permanent magnet ring 113 and the second notched ring 31 (second tunable assembly 30) and its permanent magnet ring 313 can be regarded as driven rotation members (or rotation sensing members) in a general sense, achieving frictionless power transmission through "non-contact spatial control/transmission."

The term "non-contact spatial control/transmission" here refers to control/transmission achieved without physical contact between transmission mechanisms, not through an air medium, meaning it can also be achieved in a vacuum. That is, "non-contact spatial control" is realized through non-contact forces such as electric field forces, magnetic field forces, or other field forces, without requiring a medium, and can be achieved in a vacuum. In summary, any use of field forces acting on substances placed therein to achieve "non-contact spatial control" falls within the scope of protection of the present invention. The above magnetic gears may also be referred to as magnetic wheels, magnetic drive wheels, magnetic force gears, magnetic suspension wheels, magnetic power wheels, non-contact transmission wheels, magnetic couplers, magnetic transmissions, and the like.

From the above, it can be seen that the open clamping apparatus not only includes the first notched ring 11 and the second notched ring 31 with notches but also has the first through slot 19, the second through slot 36, the third through slot 69, and the fourth through slot 76 provided on the first tunable assembly 10, the second tunable assembly 30, the linkage rod 60, and the anti-disengagement member 70, respectively. When these slots are aligned and interconnected, the intracavity medical device 80 can be conveniently inserted into the first lumen 18, the third lumen 68, and the fourth lumen 75 for clamping and can also be easily removed from the first lumen 18, the third lumen 68, and the fourth lumen 75 through the first through slot 19, the second through slot 36, the third through slot 69, and the fourth through slot 76. Additionally, when the fourth through slot 76 is misaligned with the third through slot 69 and not interconnected, it prevents the intracavity medical device 80 from accidentally disengaging from the first lumen 18, the third lumen 68, and the fourth lumen 75 during relative and/or co-directional rotation of the first tunable assembly 10 and the second tunable assembly 30. Thus, not only are clamping and removal highly convenient, but the accidental disengagement of the intracavity medical device 80 is prevented, making the delivery of the intracavity medical device 80 smoother and more reliable, improving surgical efficiency, and accommodating intracavity medical devices 80 of different sizes. Moreover, since the misalignment of the fourth through slot 76 of the anti-disengagement member 70 with the third through slot 69 of the linkage rod 60 prevents accidental disengagement of the intracavity medical device 80, it ensures the stability and smoothness of the delivery of the intracavity medical device 80. To facilitate smooth delivery of the intracavity medical device 80, a retaining structure such as a magnetic column 161 may be provided on the first support column 16 to keep the intracavity medical device 80 within the first lumen 18. Similarly, when the fourth through slot 76 of the anti-disengagement member 70 is misaligned with the third through slot 69 of the linkage rod 60, it can also keep the intracavity medical device 80 within the fourth lumen 75.

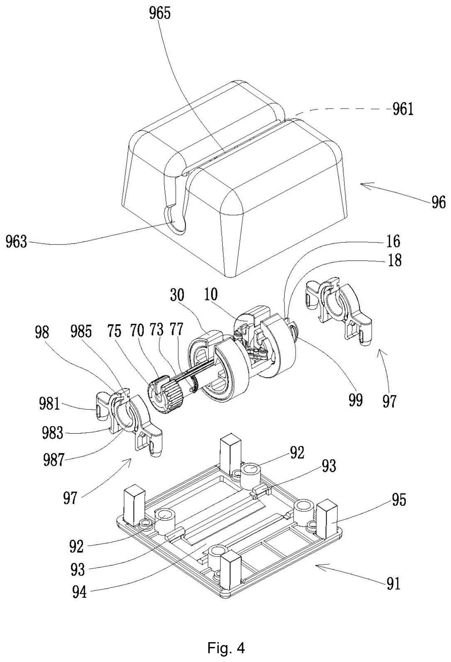

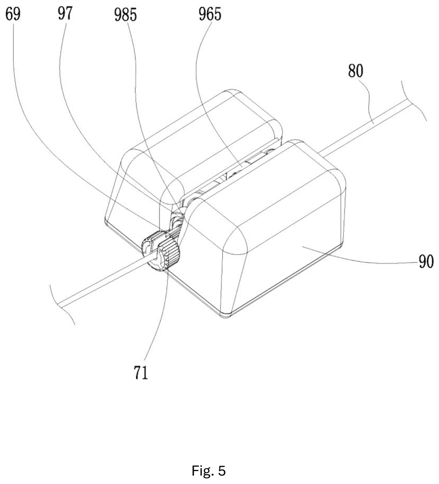

The present invention also provides an open sterile module comprising a housing, bearing seats fixed within the housing, and the open clamping apparatus supported on the bearing seats. The housing is provided with a first placement hole and a second placement hole corresponding to the first lumen and the fourth lumen of the open clamping apparatus, respectively. As shown in the embodiment of Figures 4-5, the open sterile module includes a housing 90, two bearing seats 97 fixed within the housing 90, and the above-described open clamping apparatus 100 supported on the bearing seats 97. The housing 90 includes a base plate 91 and a cover 96 that can be fixed to the base plate 91 to form a receiving space for accommodating the open clamping apparatus 100.

The base plate 91 is provided with two pairs of positioning holes 92 and two pairs of clamping blocks 93 corresponding to the two bearing seats 97. The two positioning holes 92 in each pair are aligned, and the two clamping blocks 93 in each pair are aligned. Each pair of clamping blocks 93 corresponds to a pair of positioning holes 92 and is located between the two aligned positioning holes 92 and arranged close to each other. The base plate 91 is provided with an intermediate slot 94 located between the two clamping blocks 93 of each pair. The two clamping blocks 93 of each pair are respectively located on opposite sides of the intermediate slot 94. Thus, each pair of clamping blocks 93 and the corresponding pair of positioning holes 92 are located on opposite sides of the intermediate slot 94, with the two positioning holes 92 and two clamping blocks 93 on the same side of the intermediate slot 94 being spaced apart. The edge of the base plate 91 is also provided with a magnet 95.

The cover 96 is a hollow shell, with its two opposite sides provided with a first placement hole 961 (not visible in the figures) corresponding to the first support column 16 and the first lumen 18 of the open clamping apparatus 100 and a second placement hole 963 corresponding to the sleeve portion 73 and the fourth lumen 75 of the anti-disengagement member 70. The first placement hole 961 and the second placement hole 963 are aligned. The top of the cover 96 is provided with a placement slot 965, with both ends of the placement slot 965 extending to the first placement hole 961 and the second placement hole 963 for interconnection.

Each bearing seat 97 includes a central portion 98, two elastic positioning posts 981, and two elastic hooks 983 extending from the outer edge of the central portion 98. In this embodiment, the central portion 98 is circular, provided with a slot 985 extending from the outside to a circular hole 987 at the center.

During assembly, the first support column 16 of the open clamping apparatus 100 is rotatably inserted into the circular hole 987 of the corresponding bearing seat 97 and secured with a snap ring 99 to prevent the bearing seat 97 from disengaging automatically. The anti-disengagement member 70 is removed from the third lumen 68 of the linkage rod 60 of the open clamping apparatus 100. The second end of the linkage rod 60 is rotatably inserted into the circular hole 987 of the corresponding bearing seat 97. They are then placed together above the intermediate slot 94 of the base plate 91, with the two bearing seats 97 spanning the intermediate slot 94. The positioning posts 981 of the two bearing seats 97 are inserted into the two pairs of positioning holes 92 of the base plate 91, and the two pairs of clamping blocks 93 of the base plate 91 abut against the two hooks 983 of the corresponding bearing seats 97, causing the hooks 983 to elastically deform and pass over the corresponding clamping blocks 93, thereby engaging with each other. Thus, the two bearing seats 97 are restricted from moving in opposite directions (vertically downward and upward in the figures) and fixed to the base plate 91 at intervals.

At this point, the cover 96 is placed on the base plate 91 and fixed, forming an internal space accommodating the two bearing seats 97 and the open clamping apparatus 100 supported on the bearing seats 97, with the placement slot 965 of the cover 96 communicating with the internal space of the housing 90. The first lumen 18 of the first tunable assembly 10 and the third lumen 68 of the linkage rod 60 are aligned with the first placement hole 961 and the second placement hole 963 of the cover 96, respectively. Then, the central rod 72 of the anti-disengagement member 70 is inserted through the second placement hole 963 into the third lumen 68 of the linkage rod 60, while the second end of the linkage rod 60 is inserted into the receiving groove 74, and the sleeve portion 73 passes through the second placement hole 963. When the protrusion 77 on the sleeve portion 73 abuts the edge of the circular hole 987, further pressing the twisting portion 71 causes the protrusion 77 to pass through the circular hole 987 to the opposite side edge, preventing axial movement of the bearing seat 97, allowing the central portion 98 of the bearing seat 97 to be fitted over the sleeve portion 73, with the sleeve portion 73 inserted through the second placement hole 963 and located within the circular hole 987 of the corresponding bearing seat 97, and the twisting portion 71 located outside the second placement hole 963 (i.e., outside the internal space formed by the cover 96 and the base plate 91). Thus, the first support column 16 of the open clamping apparatus 100 and the sleeve portion 73 of the anti-disengagement member 70 are rotatably positioned within the circular holes 987 of the two bearing seats 97. The slots 985 of the two bearing seats 97 are aligned on the same straight line and aligned with the placement slot 965 of the cover 96. The placement slot 965 of the cover 96 and the intermediate slot 94 of the base plate 91 are located on opposite sides of the open clamping apparatus 100.

To clamp an intracavity medical device 80, the first tunable assembly 10 and the second tunable assembly 30 of the open clamping apparatus 100 are rotated relative to each other in opposite directions as described above, i.e., the sleeve 17 of the first tunable assembly 10 rotates relative to the shaft 34 of the second tunable assembly 30, with the first support column 16 and the sleeve portion 73 rotating within the circular holes 987 of the two bearing seats 97, aligning the first through slot 19 of the first tunable assembly 10, the third through slot 69, the second through slot 36 of the second tunable assembly 30, and the slots 985 of the two bearing seats 97 on the same straight line and in communication, while also aligning with the placement slot 965 of the cover 96. As described above, the operator manually rotates the anti-disengagement member 70 to align its fourth through slot 76 with the third through slot 69 of the linkage rod 60, allowing the first lumen 18 of the first tunable assembly 10, the third lumen 68 of the linkage rod 60, and the fourth lumen 75 of the anti-disengagement member 70 to communicate with the external environment. At this point, the open clamping apparatus 100 is in an open state, allowing the intracavity medical device 80 to be inserted through the placement slot 965, the notches of the first and second tunable assemblies 10 and 30, the slots 985, the first through slot 19, the second through slot 36, the third through slot 69, and the fourth through slot 76 into the first lumen 18, the third lumen 68, and the fourth lumen 75.

The operator again manually rotates the anti-disengagement member 70 to misalign its fourth through slot 76 with the second through slot 36, the third through slot 69, and the first through slot 19, preventing communication between the fourth lumen 75 and the external environment. Then, rotating the first tunable assembly 10 and the second tunable assembly 30 relative to each other in opposite directions causes the open clamping apparatus 100 to clamp the intracavity medical device 80 for rotation, as described above, and will not be repeated here. The intermediate slot 94 provided on the base plate 91 facilitates the relative rotation of the first tunable assembly 10 and the second tunable assembly 30. Further, the open sterile module may refer to the open sterile box described in Chinese Patent Application 202310517391.3, the entire contents of which are incorporated into the present invention.

The open sterile module can be assembled with the intracavity medical device 80 during use, as described above, or pre-assembled with the intracavity medical device 80 before use or at the factory for use as an operational component of a surgical robot. Regarding the rotational drive of the first tunable assembly 10 and the second tunable assembly 30, refer to the surgical execution module and surgical robot described in Chinese Patent Application 202310565522.5, the surgical execution device and surgical robot with the same described in Chinese Patent Application 202310237481.7, the surgical robot device and its operation method described in Chinese Patent Application 202210803401.5, and the surgical robot described in Chinese Patent Application 202211105526.7, the entire contents of which are incorporated into the present invention.

The magnet 95 provided on the base plate 91 of the housing of the open sterile module allows the drive component of a surgical robot to drive the open sterile module through non-contact field forces acting on the magnet 95. In fact, any non-contact spatial control/transmission method using the field forces of the surgical robot's drive component to drive the movement of the open sterile module and the intracavity medical device 80 falls within the scope of protection of the present invention. The magnet 95 may be a permanent magnet block, an electromagnetic magnet block, or a hybrid of permanent and electromagnetic magnet blocks. The movement of the open sterile module can also be driven by other non-contact spatial control methods such as electromagnetic induction, electric field coupling, or DC resonance. In fact, any non-contact spatial control/transmission method using field forces (including magnetic field forces, electric field forces, etc.) to drive the movement of the open sterile module and the intracavity medical device 80 falls within the scope of protection of the present invention, i.e., fixing a driven movement block (or movement sensing block) in a general sense on the base plate 91. Regarding such "non-contact spatial control/transmission" methods for achieving power transmission, refer to the surgical execution module and surgical robot described in Chinese Patent Application 202310565522.5, the surgical execution device and surgical robot with the same described in Chinese Patent Application 202310237481.7, the surgical robot device and its operation method described in Chinese Patent Application 202210803401.5, and the surgical robot described in Chinese Patent Application 202211105526.7, the entire contents of which are incorporated into the present invention.

From the above, it can be seen that the housing 90 of the open sterile module has a placement slot 965, allowing the intracavity medical device 80 to be conveniently inserted from the outside into the open clamping apparatus 100 within the housing 90 for clamping and also to be easily removed. Both clamping and removal are highly convenient, and the intracavity medical device 80 is reliably held in a determined position without accidental disengagement, ensuring smooth delivery and improving surgical operation efficiency.

The embodiments described above represent only a limited number of implementations of the present invention, described in specific and detailed terms, but they should not be construed as limiting the scope of the invention's patent. It should be noted that, for those skilled in the art, several variations and improvements can be made without departing from the concept of the present invention, such as fitting discontinuous magnetic gears with notches on the outer periphery of the notched rings, all of which fall within the scope of protection of the present invention. Therefore, the scope of protection of the invention's patent is determined by the claims.

## Claims

1. A clamping apparatus of open assembly, comprising,
a first tunable assembly having a first lumen and a second tunable assembly having a second lumen are inter-connected to each other in a way that the first and second lumens are extended along the same longitudinal direction and relatively tunable; the first and second lumens are each provided with a first through slot and a second through slot, respectively, each through slot is longitudinally along respective walls of each lumen, and across from outside of the respective lumen into the respective inside of the respective lumen to allow access of an intracavity medical device to be placed into or taken out one of the lumens, wherein in the first tunable assembly, two clamping members and two corresponding pressing members are installed inside the first lumen, the two clamping members holding the intracavity medical device, wherein when the first tunable assembly is turned relative to the second tunable assembly, the two pressing members force the two clamping members to approach each other and slide into the first lumen.

2. The clamping apparatus of open assembly of claim 1, wherein the first tunable assembly comprises a first notched ring and a sleeve protruding from one side of the first notched ring, and the second tunable assembly comprises a second notched ring and a shaft protruding from one side of the second notched ring and rotatably mounted within the sleeve.

3. The clamping apparatus of open assembly of claim 2, wherein the first tunable assembly further comprises a first central body fixed along the turning axis of the first notched ring, the first central body being provided with two receiving holes for accommodating the two clamping members, the two receiving holes having a passage with the first lumen.

4. The clamping apparatus of open assembly of claim 3, wherein the first central body comprises a main body, a left block, and a right block attached together by the first notched ring, the left and right blocks being spaced apart on opposite sides of a notch of the first notched ring, and the two receiving holes being formed between the left block, the right block, and the main body.

5. The clamping apparatus of open assembly of claim 4, wherein the main body is configured with a recess to form a longitudinal section crossing the first lumen and the two receiving holes, the longitudinal section being provided with two pivot shafts, and the two pressing members being rotatably mounted on the two pivot shafts.

6. The clamping apparatus of open assembly of claim 5, wherein the left block and the right block are respectively provided with positioning caps fitted over the two pivot shafts, positioning the two pressing members between the left block, the right block, and the main body.

7. The clamping apparatus of open assembly of claim 6, wherein each of the two clamping members has a through hole having a passage with the corresponding receiving hole, each pressing member comprises a first arm extending into the receiving hole and located within the through hole of the corresponding clamping member and a second arm connected to the first arm, the first arm forming two opposing protrusions abutting against opposite sides of the corresponding through hole.

8. The clamping apparatus of open assembly of claim 7, wherein the wall of the first lumen is provided with a first flared portion, a linkage rod is disposed within the second lumen, and a first end of the linkage rod extends out of one end of the second lumen to form a large head accommodated within the first flared portion.

9. The clamping apparatus of open assembly of claim 8, wherein the second arms of the two pressing members are each provided with an elongated sliding slot, and the large head is provided with two sliding posts slidably inserted into the elongated sliding slots of the corresponding second arms.

10. The clamping apparatus of open assembly of claim 9, wherein the opposite second end of the linkage rod extends out of the opposite end edge of the second lumen and is engaged therewith, the two pressing members being two levers arranged in a snap-fit configuration; when the first tunable assembly rotates relative to the second tunable assembly, the linkage rod moves away from or toward the first tunable assembly along with the second tunable assembly, thereby causing the two pressing members to force the two clamping members to clamp or release the intracavity medical device.

11. The clamping apparatus of open assembly of claim 10, wherein the linkage rod is provided with a third lumen and a third through slot extending along the direction of the turning axis, the third through slot extending from the outside to the third lumen to allow communication with the external environment, and the third lumen being interconnected with the first lumen.

12. The clamping apparatus of open assembly of claim 11 further comprises an anti-disengagement member rotatably attached within the third lumen, along a turning axis of the anti-disengagement member, there being provided with a fourth lumen and a fourth through slot extending from the outside to the fourth lumen, whereby, the first, third, and fourth lumens extending along the same straight line and being interconnected; when the first and second tunable assemblies is turned relative to each other and the anti-disengagement member is turned to align the first, second, third, and fourth through slots, allowing the first, third, and fourth lumens to be interconnected with the external environment for placement of the intracavity medical device; when the anti-disengagement member is turned to misalign the fourth through slot with the second and third through slots, disengagement of the intracavity medical device is prevented.

13. The clamping apparatus of open assembly of claim 12, wherein the anti-disengagement member comprises a twisting portion and a central rod extending from one side of the twisting portion and rotatably inserted into the third lumen.

14. The clamping apparatus of open assembly of claim 13, wherein the anti-disengagement member further comprises a sleeve portion extending from one side of the twisting portion, a receiving groove being formed between the sleeve portion and the central rod to accommodate the opposite second end of the linkage rod, the sleeve portion being fitted over the second end of the linkage rod.

15. The clamping apparatus of open assembly of claim 8, wherein the wall of the first lumen is provided with a second flared portion near the first flared portion, and each of the two clamping members includes an abutting portion located within the second flared portion.

16. The clamping apparatus of open assembly of claim 15, wherein each of the two clamping members includes a sliding portion slidably received in the corresponding receiving hole, the through hole of the clamping member being provided in the sliding portion and extending in a direction perpendicular to an axis of the two receiving holes.

17. The clamping apparatus of open assembly of claim 4, wherein the first tunable assembly further comprises a first support column protruding from an opposite side of the first notched ring, the second tunable assembly further comprises a second support column protruding from an opposite side of the second notched ring, the first lumen and the first through slot extend through the main body, the first support column, and the sleeve, the second lumen and the second through slot extend through the second support column and the shaft, and the notches of the first and second notched rings are respectively aligned with the first and second through slots.

18. The clamping apparatus of open assembly of claim 2, wherein the first and second notched rings are configured as driven rotation members for non-contact spatial control/transmission.

19. An openable sterile module, wherein it comprises a housing and the open clamping apparatus rotatably fixed within the housing, the housing being provided with a placement slot communicating with the interior to allow an intracavity medical device to enter the housing through the placement slot and be clamped by the open clamping apparatus, the open clamping apparatus being as described in any one of claims 1 to 18.

20. The openable sterile module of claim 19, wherein opposite sides of the housing are respectively provided with a first placement hole and a second placement hole communicating with the interior of the housing, both ends of the placement slot extending to the first and second placement holes, and the first lumen of the first tunable assembly and the second lumen of the second tunable assembly being respectively aligned with the first and second placement holes.

21. The openable sterile module of claim 19, wherein the open sterile module further comprises two bearing seats fixed within the housing, the first and second tunable assemblies being tunably supported on the two bearing seats.

22. The openable sterile module of claim 19, wherein a driven movement block is fixed on the housing for non-contact spatial control/transmission.
